# EUROPÄISCHE PATENTSCHRIFT

(11) **EP 4 331 646 B1**
(45) Veröffentlichungstag und Bekanntmachung des Hinweises auf die Patenterteilung: **29.01.2025**
(21) Anmeldenummer: 23194416.6
(22) Anmeldetag: 31.08.2023
(51) Int. Cl.: A61M 5/42, A61B 5/15

(54) **VORRICHTUNG ZUR FIXIERUNG VON HAUTVENEN**
DEVICE FOR FIXING SKIN VEINS
DISPOSITIF DE FIXATION DE VEINES CUTANÉES

(30) Priorität: 31.08.2022 DE 102022122016
(43) Veröffentlichungstag der Anmeldung: 06.03.2024
(73) Patentinhaber: Reumschüssel, Jens-Arne, 22455 Hamburg (DE)
(72) Erfinder: Reumschüssel, Jens-Arne, 22455 Hamburg (DE)
(74) Vertreter: Glawe, Delfs, Moll

(56) Entgegenhaltungen:
- DE-A1- 102013 101 551
- DE-A1- 3 605 175
- US-A1- 2003 060 685
- US-A1- 2008 300 541
- US-A1- 2015 112 303
- US-A1- 2016 022 923

## Beschreibung

Die Erfindung betrifft eine Vorrichtung zur Fixierung von Hautvenen, sowie einen Satz von entsprechenden Vorrichtungen.

Bei bestimmten medizinischen Vorgängen, wie bspw. der Blutabnahme oder der intravenösen Applikation eines Medikaments, ist eine Kanüle in einer Hautvene zu platzieren, durch die dann Blut abgenommen oder ein flüssiger Wirkstoff injiziert werden kann. Bei einer Hautvene handelt es sich um eine derart unmittelbar unter der Haut eines Lebewesens liegende Vene, die - in der Regel bläulich - durch die Haut hindurchschimmert und so grundsätzlich mit bloßem Auge zu erkennen ist.

Damit die Platzierung einer Kanüle in einer Vene sicher gelingt, darf sich die Lage der Vene auch während des Einstechens der Kanüle in die Haut nicht verändern. Insbesondere wenn die Vene in einem Bereich mit lockerem Bindegewebe verläuft, verschiebt sich die Vene jedoch leicht und ist so nur schwer mit der Kanüle zu treffen. Entsprechende Venen werden auch als Rollvenen bezeichnet.

Aus Dokument DE 92 03 502 U1 ist eine Art Venenzange bekannt, mit der Rollvenen zwischen zwei Backen der Zange eingeklemmt und so fixiert werden können, dass ein Platzieren einer Kanüle in der so eingeklemmten Vene vereinfacht werden soll. Die Venenzange ist dabei aufgrund der Mechanik in der Herstellung aufwendig und auch deren Anwendung erfordert Übung und Geschick.

Dokument DE 10 2013 101 551 A1 beschreibt eine Vorrichtung für die Punktion eines Blutgefäßes mittels einer Kanüle, bei dem zwei Kufen der Vorrichtung, an dem auch die Kanüle in einer definierten Lage angeordnet ist, seitlich einer Vene positioniert werden und durch Verschieben der gesamten Vorrichtung bei Gleiten der Kufen über die Haut die Punktion durch die Kanüle erfolgt. Um dieses Gleiten zu ermöglichen, können die Kufen die Haut benachbart zur Vene nicht weit eindrücken, sodass zumindest extreme Rollvenen nicht sicher gehalten und punktiert werden können.

Aufgabe der vorliegenden Erfindung ist es Vorrichtung zur Fixierung von Hautvenen sowie einen Satz von entsprechenden Vorrichtungen zu schaffen, bei denen die aus dem Stand der Technik bekannten Nachteile nicht oder nur noch in vermindertem Umfang auftreten.

Gelöst wird diese Aufgabe durch eine Vorrichtung gemäß dem Hauptanspruch sowie einen Satz von Vorrichtungen gemäß dem nebengeordneten Anspruch. Vorteilhafte Ausführungsformen sind Gegenstand der abhängigen Ansprüche.

Demnach betrifft die Erfindung eine Vorrichtung zur Fixierung von Hautvenen zur Erleichterung der intravenösen Platzierung von Kanülen umfassend einen Haltegriff und einen sich vom Haltegriff erstreckenden Ausleger, an dessen freiem Ende eine Fixiereinheit mit entlang einer Fixierachse zweireihig angeordneten, wenigstens vier parallel zueinander verlaufenden Fixierstiften zur Fixierung einer Hautvene zwischen den beiden Reihen der Fixierstifte bei Anlage an der Haut vorgesehen ist.

Weiterhin betrifft die Erfindung einen Satz aus wenigstens zwei Ausführungsvarianten der erfindungsgemäßen Vorrichtung, wobei die wenigstens zwei Ausführungsvarianten in den freien Abständen zwischen den beiden Reihen von Fixierstiften unterschiedlich sind.

Die erfindungsgemäße Vorrichtung bietet die Möglichkeit, Hautvenen zum Platzieren von Kanülen darin zeitweise zu fixieren. Dazu wird die Vorrichtung über den Haltegriff so auf der über der Vene liegenden Haut angeordnet, dass die Fixierachse der Fixiereinheit - und damit im Bereich der Fixierstifte - parallel zur Vene verläuft und die Reihen der Fixierstift zu beiden Seiten der Vene angeordnet sind. Wird die Vorrichtung dann angedrückt, drücken die Fixierstifte die Haut zu beiden Seiten der Vene leicht ein, womit die fragliche Vene an einem seitlichen Ausweichen bzw. Rollen gehindert wird und das Platzieren einer Kanüle darin stark vereinfacht wird. Die Fixierstifte können dabei aufgrund ihrer - insbesondere im Verhältnis zu den aus dem Stand der Technik bekannten Gleitkufen - nur geringen Kontaktfläche mit der Haut bereits mit wenig Kraft bei Bedarf so weit in die Haut gedrückt werden, dass selbst schwierige Rollvenen sicher fixiert werden können. Sobald die Kanüle platziert ist, kann die Vorrichtung entfernt werden.

Da die wenigstens vier Fixierstifte zweireihig und zueinander parallel ausgebildet sein sollen, um eine Vene wie beschrieben fixieren zu können, ergibt sich zwangsläufig, dass die Fixierstifte bzw. deren Achsen unter einem Winkel zur Achse des Auslegers, an welchem die Fixierstifte angeordnet sind, verlaufen. Weiterhin ergibt sich aus der beschriebenen Verwendung, dass die freien Enden der Fixierstifte in der Regel in einer gemeinsamen Ebene angeordnet sind, die bei der ordnungsgemäßen Verwendung im Wesentlichen parallel zur Fixierachse verläuft, um so die Fixierung der Vene durch die Fixierstifte sicherzustellen.

Das kurzzeitige leichte Eindrücken der Haut zur Fixierung einer Vene erfolgt grundsätzlich irritationsfrei, d. h. weder treten beim leichten Eindrücken Schmerzen auf noch kommt es zu Beschädigungen der Haut oder des darunter liegenden Gewebes.

Da die erfindungsgemäße Vorrichtung vollständig ohne eine Mechanik auskommt, ist ihre Herstellung sehr einfach und kostengünstig möglich. Insbesondere ist es möglich, dass die Vorrichtung einstückig ausgeführt und bspw. aus Kunststoff ist.

Es ist bevorzugt, wenn die Fixierachse parallel zu einer Längserstreckung des Auslegers verläuft. Bei einer entsprechenden Ausgestaltung des Auslegers mit einer ersichtlichen Längserstreckung und einer Anordnung der Fixierachse parallel dazu, dient der Ausleger unmittelbar als optisches Hilfsmittel zur korrekten Positionierung der Vorrichtung bei deren Verwendung. Die konkrete Ausgestaltung des Auslegers ist dabei beliebig, sofern denn eine konkrete Längserstreckung erkennbar ist.

Unabhängig davon kann der Ausleger bevorzugt zweigeteilt ausgestaltet sein, sodass jeweils eine Reihe der Fixierstifte an einem der beiden Teile des Auslegers angeordnet ist. In der Folge ergibt sich ein Spalt zwischen den beiden Auslegerspalten, der grundsätzlich parallel zur Fixierachse verläuft. Der Spalt kann dann als optische Ausrichtungshilfe dienen und ermöglicht zusätzlich einen unmittelbaren Blick auf eine mit der Vorrichtung fixierte Vene. Je nach Ausgestaltung des Auslegers und/oder dessen Material ist es auch möglich, durch plastische Verformung durch Biegen wenigstens eines Teils des Auslegers die Breite des Spalts und damit den freien Abstand zwischen den beiden Reihen an Fixierstiften zu verändern, um so die Fixierwirkung der Vorrichtung im Hinblick auf eine bestimmte Vene verbessern zu können.

Es ist bevorzugt, wenn der Ausleger und/oder die Fixierstifte aus einem transparenten oder transluzenten Material sind. Sind Ausleger und/oder Fixierstifte entsprechend ausgestaltet, kann ein ggf. ungünstiger Schattenwurf im Bereich der Fixierstifte bei der Verwendung der Vorrichtung vermieden oder zumindest deutlich reduziert werden. Ist die Vorrichtung einstückig ausgeführt, ist dann auch der Haltegriff transparent oder transluzent.

Vorzugsweise umfasst der Haltegriff ein zum Fassen und Halten per Pinzettengriff ausgestaltete Haltefläche. Ein Halten der Vorrichtung per Pinzettengriff ist für die ordnungsgemäße Verwendung ausreichend. Indem der Haltegriff auf den Pinzettengriff ausgelegt ist, kann der Haltegriff im Wesentlichen auf die dafür erforderliche, vorzugsweise maximal fingerkuppengroße Haltefläche beschränkt sein. Ist die Haltefläche elliptisch ausgestaltet, können die Halbachsen bspw. 0,9 mm und 0,6 mm betragen.

Alternativ zu einer zum Fassen und Halten per Pinzettengriff ausgestaltete Haltefläche kann der Haltegriff auch eine Hülse umfassen, die zum Hineinstecken eines menschlichen Fingers ausgebildet ist und sich vergleichbar einem Ring oder einem Fingerhut an einen hineingesteckten Finger anlegen kann, um so eine Verbindung zu schaffen. Indem die Vorrichtung auf diese Weise mit einem Finger verbindbar ist, kann die Verwendung der Vorrichtung vereinfacht werden. Die Hülse kann rohrförmig oder ringförmig ausgestaltet sein. Die Hülse kann beidseitig offen sein, insbesondere im Falle einer rohrförmigen Ausgestaltung ist es aber auch möglich, dass eine Seite der Hülse geschlossen ist.

Die Hülse kann einstückig mit der Vorrichtung ausgebildet sein. Es ist aber auch möglich, dass die Hülse getrennt von der übrigen Vorrichtung ausgebildet ist und bspw. per Rastverbindung bei Bedarf mit der Vorrichtung verbindbar ist. Insbesondere ist es möglich, dass die Hülse für eine Rastverbindung mit einer an der Vorrichtung vorgesehenen Haltefläche ausgebildet ist. Die Vorrichtung lässt sich dann wahlweise per Pinzettengriff halten und - nach Befestigung einer Hülse daran - auf einen Finger aufstecken.

Es ist bevorzugt, wenn die Haltefläche des Haltegriffs und/oder die Hülse unter einem Winkel gegenüber eine Längserstreckung des Auslegers angeordnet ist. Ist die Haltefläche unter einem Winkel angeordnet, lässt sich auch bei per Pinzettengriff gefasster Haltefläche häufig eine nahezu parallele Ausrichtung des Auslegers zu der Vene, die fixiert werden soll, erreichen. Vergleichbares gilt im Falle einer Hülse. Der Winkel beträgt dabei vorzugsweise wenigstens 10°, weiter vorzugsweise wenigstens 20°.

Es ist bevorzugt, wenn die Haltefläche des Handgriffs im Wesentlichen nur zu einer Seite der Längserstreckung des Auslegers angeordnet ist. Gegenüber dem Ausleger erstreckt sich der Handgriff also im Wesentlichen nur in eine senkrecht zur Längserstreckung verlaufende Richtung. Dadurch kann bei dem Platzieren von Kanülen in einer Vene typischerweise vorhandenen Lichtverhältnissen ein möglicherweise ungünstiger Schattenwurf der die Vorrichtung haltenden Hand häufig vermieden werden. Es können für die Verwendung von Links- und Rechtshändern unterschiedliche Ausgestaltung der Vorrichtung vorgesehen sein, bei denen sich der Handgriff jeweils in unterschiedliche Richtungen gegenüber der Längserstreckung des Auslegers erstreckt. Ist eine Hülse vorgesehen, ist diese vorzugsweise zentral gegenüber der Achse des Auslegers angeordnet. Auch wenn bereits vier Fixierstifte an der Fixiereinheit ausreichen können, um eine Vene zu fixieren, ist es bevorzugt, wenn die Fixiereinheit insgesamt sechs oder acht Fixierstifte umfasst, womit sich dann jeweils zwei Reihen von drei oder vier Fixierstiften ergeben. Eine entsprechende Anzahl von Fixierstiften haben sich für die meisten Anwendungsfälle als ausreichend erwiesen. Besonders vorteilhaft hat sich dabei eine Ausführungsform mit sechs Fixierstiften erwiesen.

Die Fixierstifte können einen beliebigen Querschnitt aufweisen. Bevorzugt ist es aber, wenn der Querschnitt eine kreisrunde Form aufweist.

Es ist bevorzugt, wenn der freie Abstand zwischen den beiden Reihen von Fixierstiften 1 mm bis 5 mm, vorzugsweise 1 mm bis 2 mm, 2 mm bis 3 mm oder 3 mm bis 4 mm, weiter vorzugsweise 1,5 mm, 2,5 mm oder 3,5 mm beträgt. Die entsprechenden Abstände sind jeweils bei einer Vielzahl von Hautvenen für deren Fixierung geeignet.

Dabei ist zu berücksichtigen, dass Hautvenen unterschiedliche Durchmesser bzw. Breiten aufweisen können. Um eine ordnungsgemäße Fixierung von Hautvenen unterschiedlicher Durchmesser sicherstellen zu können, betrifft die Erfindung auch einen Satz von erfindungsgemäßen Vorrichtungen. Dabei sind die Vorrichtungen des Satzes von wenigstens zwei Ausführungsvarianten, die sich wenigstens in den freien Abständen zwischen den beiden Reihen von Fixierstiften unterscheiden. Zur Fixierung einer Vene kann dann diejenige Vorrichtung aus dem Satz an Vorrichtungen gewählt werden, deren freier Abstand zwischen den Fixierstiften größer ist als der Durchmesser bzw. die Breite der Vene, aber dennoch möglichst nah an diesem bzw. dieser liegt. Mit ersterer Bedingung wird sichergestellt, dass die Vorrichtung bei ordnungsgemäßer Verwendung nicht mit einem ihrer Fixierstifte auf die Vene selbst drückt; mit letzterer Bedingung wird sichergestellt, dass die Vene auch tatsächlich ausreichend fixiert wird und sich nicht etwas zwischen den Reihen von Fixierstiften noch bewegen kann.

Die freien Abstände zwischen den beiden Reihen der Fixierstifte können bei den einzelnen Ausführungsformen der Vorrichtung des Satzes vorzugsweise 1 mm bis 2 mm, 2 mm bis 3 mm oder 3 mm bis 4 mm, weiter vorzugsweise 1,5 mm, 2,5 mm oder 3,5 mm betragen.

Es ist bevorzugt, wenn der Satz Vorrichtungen in wenigstens drei Ausführungsvarianten umfasst, wobei die drei Ausführungsvarianten dabei die vorstehend aufgeführten freien Abstände zwischen den beiden Reihen der Fixierstifte aufweisen können. Mit einem Satz umfassend diese freien Abstände kann ein Großteil der Hautvenen von Menschen fixiert werden.

Alternativ oder zusätzlich ist es möglich, dass der Satz Vorrichtungen umfasst, bei denen die Halteflächen zu unterschiedlichen Seiten gegenüber der Längserstreckung des jeweiligen Auslegers angeordnet sind oder Hülsen mit unterschiedlichen Durchmessern vorgesehen sind. Der Satz umfasst dann Vorrichtungen, die jeweils besonders für Rechts- und Linkshänder bzw. für unterschiedlich große Finger geeignet sind.

Die Erfindung wird nun anhand vorteilhafter Ausführungsformen unter Bezugnahme auf die beigefügten Zeichnungen beispielhaft beschrieben. Es zeigen:
- Figur 1:: eine schematische Darstellung einer ersten Ausführungsform einer erfindungsgemäßen Vorrichtung;
- Figur 2:: eine schematische Darstellung einer ersten Ausführungsform eines erfindungsgemäßen Satzes aus erfindungsgemäßen Vorrichtungen;
- Figur 3:: eine schematische Darstellung einer Ausführungsalternative zur Vorrichtung gemäß Figur 1; und
- Figur 4:: eine schematische Darstellung einer zweiten Ausführungsform einer erfindungsgemäßen Vorrichtung.

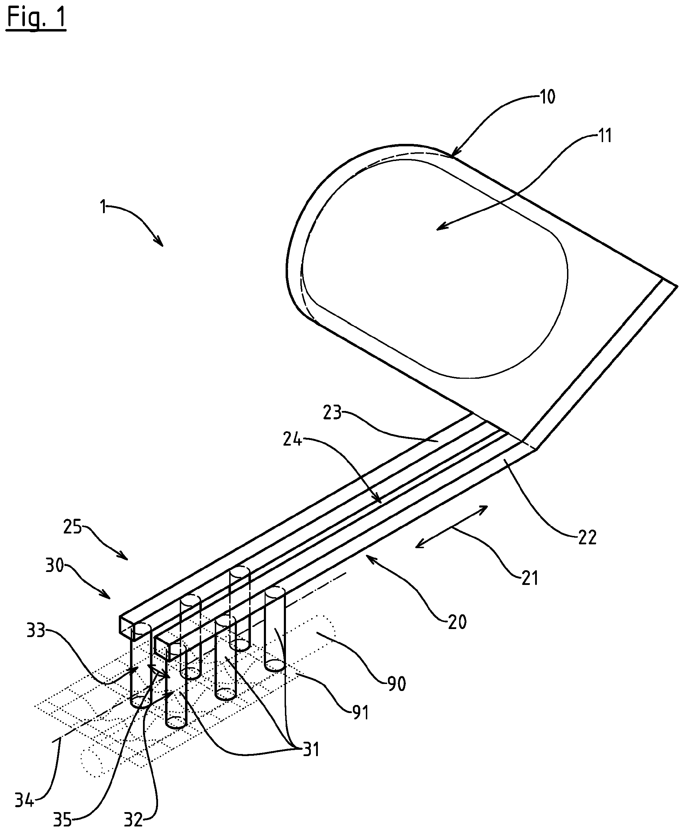

In Figur 1 ist ein erstes Ausführungsbeispiel einer erfindungsgemäßen Vorrichtung 1 schematisch dargestellt. Auch ist deren Funktionsweise bei Verwendung skizziert.

Die Vorrichtung 1, die einstückig aus transparentem Kunststoff gefertigt ist, umfasst einen Haltegriff 10, einen sich davon erstreckenden Ausleger 20 und eine am freien Ende 21 des Auslegers 20 angeordnete Fixiereinheit 30.

Der Haltegriff 10 ist zum Greifen per Pinzettengriff ausgebildet und umfasst dafür eine geeignet ausgestattete Haltefläche 11, deren Ausdehnung im Wesentlichen der durchschnittlichen Größe einer menschlichen Fingerkuppe entspricht. Der Haltegriff 10 selbst ist nur geringfügig größer als die Haltefläche 11.

An einer Seite des Haltegriffs 10 ist unter einem Winkel von ca. 25° der Ausleger 20 angeordnet, der sich in die Längsrichtung - angedeutet durch den Doppelpfeil 21 - erstreckt. Ausgehend vom Ausleger 20 ist die Haltefläche 11 des Haltegriffs 10 also unter einem Winkel von ca. 25° angeordnet und erstreckt sich im Wesentlichen nur zu einer Seite der Längsrichtung 21 des Auslegers 20.

Der Ausleger 20 ist in Längsrichtung 21 in zwei Teile 22, 23 aufgeteilt ausgeführt, sodass sich ein in Längsrichtung 21 erstreckender Spalt 24 ergibt. Im Ausgangstand weist der Spalt 24 eine vorgegebene Breite auf; durch Biegen wenigstens einer der beiden Teile 22, 23 des Auslegers 20 in Richtung quer zur Längsrichtung 21, sodass sich eine plastische Verformung des transparenten Kunststoffs, aus dem die gesamte Vorrichtung 1 und somit auch der Ausleger 20 ist, ergibt, kann die Breite des Spalts 24 insbesondere im Bereich des freien Endes 25 des Auslegers 20 aber auch noch leicht verändert werden. Wie unmittelbar aus der Figur 1 ersichtlich, verlaufen die Fixierstifte 31 senkrecht zur Längsrichtung 21 des Auslegers 20; außerdem liegen die freien Enden der Fixierstifte 31 in einer gemeinsamen Ebene, die parallel zur Fixierachse 34 verläuft.

Am freien Ende 25 des Auslegers 20 ist die Fixiereinheit 30 vorgesehen. Die Fixiereinheit 30 umfasst dabei sechs zueinander parallel verlaufende Fixierstifte 31, die zweireihig in den beiden Reihen 32, 33 zu beiden Seiten der zentral dazwischen verlaufenden Fixierachse 34 angeordnet sind. Die Fixierachse 34 verläuft dabei parallel zur Längsrichtung 21 des Auslegers 20, wobei die Fixierstifte 31 der einen Reihe 32 an dem einen Teil 22 des Auslegers 20 angeordnet sind, während die Fixierstifte 31 der anderen Reihe 33 an dem anderen Teil 23 des Auslegers 20 angeordnet sind.

Zwischen den beiden Reihen 32, 33 an Fixierstiften 31 ergibt sich ein freier Abstand 35 der im Ausgangszustand der Vorrichtung 1 1,5 mm, durch das oben beschrieben Biegen der Teile 22, 23 des Auslegers 20 aber entsprechend der Veränderung der Breite des Spalts 24 bei Bedarf im Bereich ± 0,5 mm noch nachjustiert werden kann.

In Figur 1 ist auch die Verwendung der Vorrichtung 1 skizziert: Zur Fixierung einer Vene 90, die unmittelbar unter der Haut 91 eines Lebewesens angeordnet ist und daher durch die Haut 91 - in der Regel bläulich - durchschimmert, wird die am Haltegriff 10 per Pinzettengriff gehaltenen (nicht dargestellt) Vorrichtung 1 derart auf der Haut 91 angeordnet, dass die Vene 90 im Bereich der Fixiereinheit 30 im wesentliche parallel zur Fixierachse 34 und insbesondere zwischen den beiden Reihen 32, 33 an Fixierstiften 31 verläuft. Aufgrund der transparenten Ausgestaltung der Vorrichtung 1 sowie des Spalts 24 im Ausleger 20 ist die ordnungsgemäße Anordnung bei für ein Platzieren einer Kanüle in der Vene 90 üblicherweise vorgesehenen Beleuchtung grundsätzlich gut zu überprüfen.

Ist die Vorrichtung 1 ordnungsgemäß gegenüber der Vene 90 angeordnet, können durch Aufbringen entsprechender Kraft am Haltegriff 10 der Vorrichtung 1 die Fixierstifte 31 leicht in die Haut 91 eingedrückt werden, sodass sich die Haut 91 - wie in Figur 1 angedeutet - im Bereich der Fixierstifte 31 verformt. Das Eindrücken ist dabei derart leicht, dass grundsätzlich keine Schmerzen auftreten oder die Haut 91 im Bereich der Fixierstifte 31 durch diese beschädigt würde.

Durch das Eindrücken der Fixierstifte 31 in die Haut 91 wird die Vene 90 derart fixiert, dass sie auch beim Platzieren einer Kanüle darin zumindest nicht mehr seitlich "wegrollen" kann. Die Vorrichtung 1 erleichtert so das Platzieren einer Kanüle in der Vene 90, bspw. um Blut abzunehmen oder ein Medikament intravenös zu verabreichen.

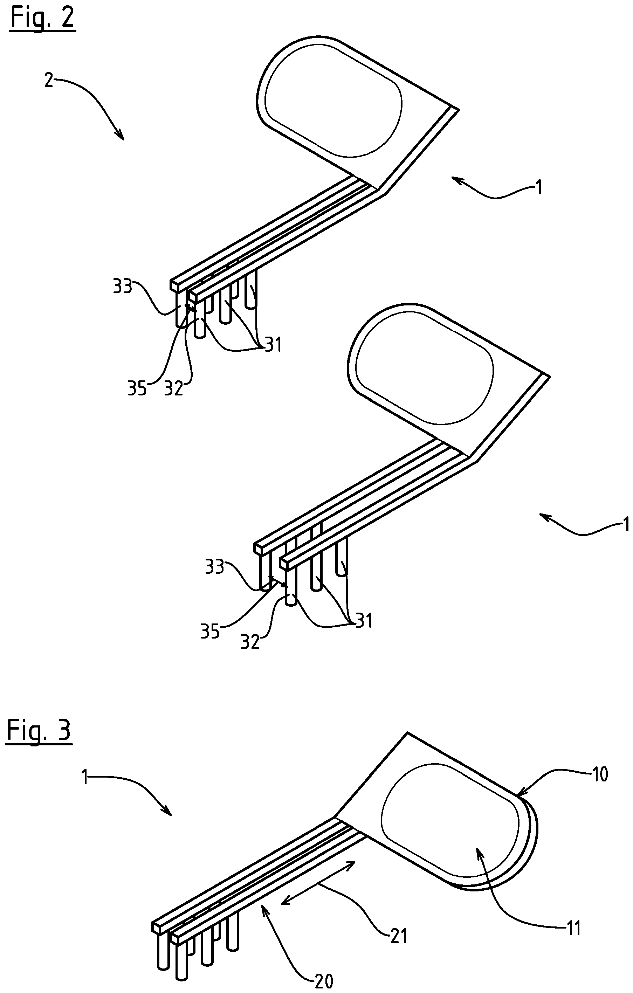

In Figur 2 ist ein erfindungsgemäßer Satz 2 erfindungsgemäßer Vorrichtungen 1 gezeigt. Die beiden Vorrichtungen 1 sind dabei grundsätzlich gemäß Figur 1 ausgebildet, weshalb auf die diesbezüglichen Erläuterungen verwiesen wird.

Der Unterschied zwischen den beiden Vorrichtungen 1 des Satzes 2 liegt in unterschiedlichen freien Abständen 35 zwischen den jeweils beiden Reihen 32, 33 der Fixierstifte 31. Dieser Abstand beträgt bei der einen Vorrichtung 1 1,5 mm, bei der anderen Vorrichtung 3,5 mm. Der Satz 2 kann auch noch ergänzt werden durch eine weitere erfindungsgemäße Vorrichtung 1, bei der der genannte Abstand 35 2,5 mm beträgt (nicht dargestellt).

Für die Fixierung einer Vene 90 (vgl. Figur 1) kann aus dem Satz 2 an Vorrichtungen 1 diejenige ausgewählt werden, bei welcher der freie Abstand 35 zwischen den jeweils beiden Reihen 32, 33 der Fixierstifte 31 größer ist als der Durchmesser bzw. die Breite der zu fixierenden Vene 90, wobei das Übermaß jedoch möglichst gering sein soll, um eine möglichst gute Fixierung der Vene 90 sicherzustellen.

In Figur 3 ist eine Ausführungsalternative zur Vorrichtung 1 gemäß Figur 1 gezeigt.

Bei dieser Ausführungsalternative ist die Seite, zu der die Haltefläche 11 des Handgriffs 10 gegenüber der Längserstreckung 21 des Auslegers 20 angeordnet ist, im Vergleich zur Ausführungsvariante gemäß Figur 1 die Gegenüberliegende. Dadurch wird eine alternative Handhaltung bei der Verwendung der Vorrichtung 1 gemäß Figur 3 im Vergleich zu der Vorrichtung 1 gemäß Figur 1 ermöglicht - sei es bspw. die Verwendung der linken statt der rechten Hand oder aber ein "Vertauschen" der beiden Finger beim Pinzettengriff. Der Benutzer kann die für ihn angenehmste Haltung einer erfindungsgemäßen Vorrichtung 1 ermitteln, bei der gleichzeitig noch die Beschattung des Bereichs, in dem die Kanüle platziert werden soll, möglichst gering ist.

Es ist möglich, dass die Ausführungsalternative der Vorrichtung gemäß Figur 3 Teil eines Satzes gemäß Figur 2 ist. Es ist bspw. denkbar, dass der Satz 2 drei Ausführungsvarianten der Vorrichtung 1 mit jeweils unterschiedlichen freien Abständen 35 zwischen den zwei Reihen 32, 33 an Fixierstiften 31, wie sie in Zusammenhang mit Figur 2 beschrieben sind, und einer Anordnung der Haltefläche 11 gemäß Figur 1 ebenso umfasst, wie drei weitere Ausführungsvarianten der Vorrichtung 1 mit vergleichbaren Abständen 35, jedoch einer Anordnung der Haltefläche 11 gemäß Figur 3.

Bei einem solchen Satz 2 ist eine erhöhte Flexibilität bei der Verwendung einer Vorrichtung 1 daraus gegeben.

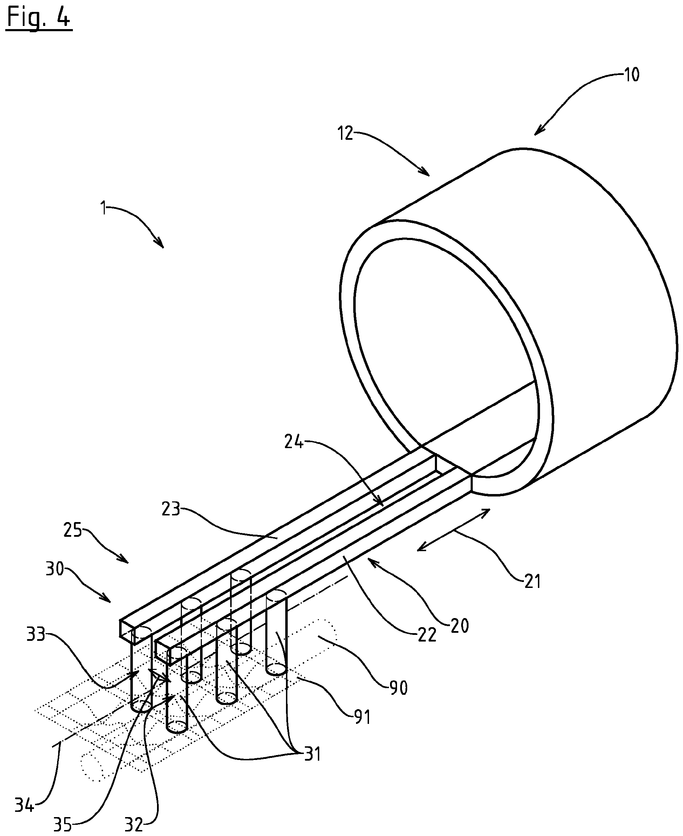

In Figur 4 ist ein zweites Ausführungsbeispiel einer erfindungsgemäßen Vorrichtung 1 gezeigt, welches abgesehen von der Ausgestaltung des Haltegriffs 10 mit denjenigen aus Figuren 1 bis 3 übereinstimmt, sodass auf die obigen Erläuterungen verwiesen und im folgenden nur auf die abweichende Ausgestaltung des Haltegriffs 10 eingegangen wird.

Der Haltegriff 10 ist bei dem Ausführungsbeispiel gemäß Figur 4 als Hülse 12 ausgeführt, in die ein Benutzer der Vorrichtung 1 einen Finger einstecken kann. Die Hülse 12 ist grundsätzlich rohrförmig ausgestaltet, wobei die Länge der Hülse 12 auch derart gering gewählt sein kann, dass sich eine Ringform ergibt.

Im dargestellten Ausführungsbeispiel ist die Hülse 12 beidseitig offen. Es ist aber auch möglich, dass die Hülse 12 an der dem Ausleger 20 näherliegenden Seite geschlossen ist, sodass sich eine mit einem Fingerhut vergleichbare Ausgestaltung ergibt.

Ist vergleichbar zu Figuren 2 und 3 ein Satz aus erfindungsgemäßen Vorrichtungen 1 vorgesehen, so kann bei einer Ausgestaltung gemäß Figur 4 als zusätzliche Varianz noch der Winkel der Hülse 12 gegenüber dem Ausleger 20 und/oder der Durchmesser der Hülse 12 berücksichtigt werden. Mit Erstem lasen sich verschiedene Anwendungsvarianten der Vorrichtung 1, bspw., an welchem Finger welcher Hand die Vorrichtung 1 aufgesteckt wird, mit Letzterem unterschiedlichen Fingergrößen abdecken.

## Patentansprüche

1. Vorrichtung (1) zur Fixierung von Hautvenen (90) zur Erleichterung der intravenösen Platzierung von Kanülen umfassend einen Haltegriff (10) und einen sich vom Haltegriff (10) erstreckenden Ausleger (20),
**dadurch gekennzeichnet dass**,
an das freie Ende (25) des Auslegers (20) eine Fixiereinheit (30) mit entlang einer Fixierachse (34) zweireihig angeordneten, wenigstens vier parallel zueinander verlaufenden Fixierstiften (31) zur Fixierung einer Hautvene (90) zwischen den beiden Reihen (32, 33) der Fixierstifte (31) bei Anlage an der Haut (91) vorgesehen ist.

2. Vorrichtung nach Anspruch 1,
**dadurch gekennzeichnet, dass**
die Fixierachse (34) parallel zu einer Längserstreckung (21) des Auslegers (20) verläuft.

3. Vorrichtung nach einem der vorhergehenden Ansprüche,
**dadurch gekennzeichnet, dass**
der Ausleger (20) derart zweigeteilt ausgestaltet ist, dass jeweils eine Reihe (32, 33) der Fixierstifte (31) an einem der beiden Teile (22, 23) des Auslegers (20) angeordnet ist.

4. Vorrichtung nach einem der vorhergehenden Ansprüche,
**dadurch gekennzeichnet, dass**
der Ausleger (20) und/oder die Fixierstifte (31) aus einem transparenten oder transluzenten Material sind.

5. Vorrichtung nach einem der vorhergehenden Ansprüche,
**dadurch gekennzeichnet, dass**
der Haltegriff (10) eine zum Fassen per Pinzettengriff ausgestaltete Haltefläche (11) umfasst.

6. Vorrichtung nach Anspruch 5,
**dadurch gekennzeichnet, dass**
die Haltefläche (11) des Haltegriffs (10) im Wesentlichen nur zu einer Seite einer Längserstreckung (21) des Auslegers (20) angeordnet ist.

7. Vorrichtung nach einem der Ansprüche 1 bis 4,
**dadurch gekennzeichnet, dass**
der Haltegriff (10) eine Hülse (12) umfasst, die zum Hineinstecken eines menschlichen Fingers ausgebildet ist.

8. Vorrichtung nach Anspruch 5 bis 7,
**dadurch gekennzeichnet, dass**
die Haltefläche (11) oder die Hülse (12) des Haltegriffs (10) unter einem Winkel von wenigstens 10° gegenüber einer Längserstreckung (21) des Auslegers (20) angeordnet ist.

9. Vorrichtung nach einem der vorhergehenden Ansprüche,
**dadurch gekennzeichnet, dass**
sechs oder acht Fixierstifte (31) vorgesehen sind.

10. Vorrichtung nach einem der vorhergehenden Ansprüche,
**dadurch gekennzeichnet, dass**
der freie Abstand (35) zwischen den beiden Reihen (32, 33) von Fixierstiften (31) 1 mm bis 5 mm, vorzugsweise 1 mm bis 2 mm, 2 mm bis 3 mm oder 3 mm bis 4 mm, weiter vorzugsweise 1,5 mm, 2,5 mm oder 3,5 mm beträgt.

11. Satz (2) aus wenigstens zwei Ausführungsvarianten der Vorrichtung (1) gemäß einem der vorhergehenden Ansprüche, wobei die wenigstens zwei Ausführungsvarianten in den freien Abständen (35) zwischen den beiden Reihen (32, 33) von Fixierstiften (31) unterschiedlich sind.

12. Satz nach Anspruch 11,
**dadurch gekennzeichnet, dass**
die freien Abstände (35) zwischen den beiden Reihen (32, 33) der Fixierstifte (31) bei den einzelnen Ausführungsformen der Vorrichtung (1) des Satzes (2) vorzugsweise 1 mm bis 2 mm, 2 mm bis 3 mm oder 3 mm bis 4 mm, weiter vorzugsweise 1,5 mm, 2,5 mm oder 3,5 mm betragen.

13. Satz nach Anspruch 11 oder 12,
**dadurch gekennzeichnet, dass**
der Satz (3) Vorrichtung (1) in wenigstens drei Ausführungsvarianten umfasst.

14. Satz nach einem der Ansprüche 11 bis 13,
**dadurch gekennzeichnet, dass**
der Satz (3) Vorrichtungen (1) umfasst, bei denen die Halteflächen (11) zu unterschiedlichen Seiten gegenüber der Längserstreckung (21) des jeweiligen Auslegers (20) angeordnet sind, oder bei denen Hülsen (12) mit unterschiedlichen Durchmessern vorgesehen sind.

## Claims

1. Device (1) for fixing skin veins (90) to facilitate the intravenous placing of cannulas, comprising a handle (10) and a cantilever arm (20), which extends from the handle (10), **characterized in that** a fixing unit (30) with at least four mutually parallel fixing pins (31), arranged in two rows along a fixing axis (34), for fixing a skin vein (90) between the two rows (32, 33) of the fixing pins (31) when placed on the skin (91) is provided at the free end (25) of the cantilever arm (20).

2. Device according to Claim 1, **characterized in that** the fixing axis (34) runs parallel to a longitudinal extent (21) of the cantilever arm (20).

3. Device according to one of the preceding claims, **characterized in that** the cantilever arm (20) is of two-part configuration in such a way that in each case one row (32, 33) of the fixing pins (31) is arranged on one of the two parts (22, 23) of the cantilever arm (20) .

4. Device according to one of the preceding claims, **characterized in that** the cantilever arm (20) and/or the fixing pins (31) is/are made from a transparent or translucent material.

5. Device according to one of the preceding claims, **characterized in that** the handle (10) comprises a holding surface (11) configured for grasping and holding in a pincer grip.

6. Device according to Claim 5, **characterized in that** the holding surface (11) of the handle (10) is arranged substantially only on one side of a longitudinal extent (21) of the cantilever arm (20).

7. Device according to one of Claims 1 to 4, **characterized in that** the handle (10) comprises a sleeve (12) which is designed to allow a human finger to be inserted.

8. Device according to Claim 5 to 7, **characterized in that** the holding surface (11) or the sleeve (12) of the handle (10) is arranged at an angle of at least 10° relative to a longitudinal extent (21) of the cantilever arm (20).

9. Device according to one of the preceding claims, **characterized in that** six or eight fixing pins (31) are provided.

10. Device according to one of the preceding claims, **characterized in that** the clearance (35) between the two rows (32, 33) of fixing pins (31) is 1 mm to 5 mm, preferably 1 mm to 2 mm, 2 mm to 3 mm or 3 mm to 4 mm, as a further preference 1.5 mm, 2.5 mm or 3.5 mm.

11. Set (2) of at least two variant embodiments of the device (1) according to one of the preceding claims, wherein the at least two variant embodiments differ in the clearances (35) between the two rows (32, 33) of fixing pins (31).

12. Set according to Claim 11, **characterized in that** the clearances (35) between the two rows (32, 33) of the fixing pins (31) in the individual embodiments of the device (1) in the set (2) are preferably 1 mm to 2 mm, 2 mm to 3 mm or 3 mm to 4 mm, as a further preference 1.5 mm, 2.5 mm or 3.5 mm.

13. Set according to Claim 11 or 12, **characterized in that** the set (3) comprises devices (1) in at least three variant embodiments.

14. Set according to one of Claims 11 to 13, **characterized in that** the set (3) comprises devices (1) in which the holding surfaces (11) are arranged on different sides of the longitudinal extent (21) of the respective cantilever arm (20), or in which sleeves (12) with different diameters are provided.

## Revendications

1. Dispositif (1) de fixation de veines cutanées (90) destiné à faciliter la pose de canules par voie intraveineuse, ledit dispositif comprenant un élément de préhension (10) et un bras (20) qui s'étend depuis l'élément de préhension (10),
**caractérisé en ce que**
une unité de fixation (30), pourvue d'au moins quatre broches de fixation (31), disposées sur deux rangées le long d'un axe de fixation (34) et s'étendant parallèlement les unes aux autres, étant prévues jusqu'à l'extrémité libre (25) du bras (20) entre les deux rangées (32, 33) de broches de fixation (31), lorsqu'elle vient en appui sur la peau (91), afin de fixer une veine cutanée (90).

2. Dispositif selon la revendication 1,
**caractérisé en ce que**
l'axe de fixation (34) s'étend parallèlement à une extension longitudinale (21) du bras (20).

3. Dispositif selon l'une des revendications précédentes,
**caractérisé en ce que**
le bras (20) est conçu en deux parties de manière à ce qu'une rangée (32, 33) de broches de fixation (31) soit disposée sur l'une des deux parties (22, 23) du bras (20) .

4. Dispositif selon l'une des revendications précédentes,
**caractérisé en ce que**
le bras (20) et/ou les broches de fixation (31) sont en un matériau transparent ou translucide.

5. Dispositif selon l'une des revendications précédentes,
**caractérisé en ce que**
le bras (10) comprend une surface de retenue (11) conçue pour être saisie avec un préhenseur à pincettes.

6. Dispositif selon la revendication 5,
**caractérisé en ce que**
la surface de retenue (11) de l'élément de préhension (10) est disposée sensiblement uniquement sur un côté d'une extension longitudinale (21) du bras (20).

7. Dispositif selon l'une des revendications 1 à 4,
**caractérisé en ce que**
l'élément de préhension (10) comprend un manchon (12) conçu pour insérer un doigt humain.

8. Dispositif selon les revendications 5 à 7,
**caractérisé en ce que**
la surface de retenue (11) ou le manchon (12) de l'élément de préhension (10) est disposée suivant un angle d'au moins 10° par rapport à une extension longitudinale (21) du bras (20).

9. Dispositif selon l'une des revendications précédentes,
**caractérisé en ce que**
six ou huit broches de fixation (31) sont prévues.

10. Dispositif selon l'une des revendications précédentes,
**caractérisé en ce que**
la distance libre (35) entre les deux rangées (32, 33) de broches de fixation (31) est de 1 mm à 5 mm, de préférence de 1 mm à 2 mm, de 2 mm à 3 mm ou de 3 mm à 4 mm, plus préférablement de 1,5 mm, 2,5 mm ou 3,5 mm.

11. Ensemble (2) d'au moins deux variantes de réalisation du dispositif (1) selon l'une des revendications précédentes, les au moins deux variantes de réalisation se différenciant par les distances libres (35) entre les deux rangées (32, 33) de broches de fixation (31).

12. Ensemble selon la revendication 11,
**caractérisé en ce que**
les distances libres (35) entre les deux rangées (32, 33) de broches de fixation (31) dans les différents modes de réalisation du dispositif (1) de l'ensemble (2) sont de préférence de 1 mm à 2 mm, de 2 mm à 3 mm ou 3 mm à 4 mm, plus préférablement de 1,5 mm, 2,5 mm ou 3,5 mm.

13. Ensemble selon la revendication 11 ou 12,
**caractérisé en ce que**
l'ensemble (3) comprend le dispositif (1) dans au moins trois variantes de réalisation.

14. Ensemble selon l'une des revendications 11 à 13,
**caractérisé en ce que**
l'ensemble (3) comprend des dispositifs (1) dans lesquels les surfaces de retenue (11) sont disposées sur des côtés différents par rapport à l'extension longitudinale (21) du bras (20) respectif, ou dans lesquels des manchons (12) de diamètres différents sont prévus.
